# FASCICULE DE BREVET EUROPEEN

(11) **EP 1 075 824 B1**
(45) Date de publication et mention de la délivrance du brevet: **09.01.2008**
(21) Numéro de dépôt: 00400708.4
(22) Date de dépôt: 15.03.2000
(51) Int. Cl.: A61F 2/06

(54) **Structure d'une prothèse destinée à être implantée dans un conduit humain ou animal**
Prothesenanordnung zur Implantation in ein menschliches oder tierisches Gefäss
Prosthesis structure for implanting in a human or animal vessel

(30) Priorité: 09.08.1999 FR 9910318
(43) Date de publication de la demande: 14.02.2001
(73) Titulaire: Novatech SA, 13705 La Ciotat Cedex (FR)
(72) Inventeur: Fedida, José, 06370 Mouans-Sartoux (FR)
(74) Mandataire: Hauer, Bernard

(56) Documents cités:
- EP-A- 0 691 108
- EP-A- 0 749 729
- EP-A- 0 808 612
- WO-A-95/21592
- WO-A-99/44536
- FR-A- 2 745 172
- US-A- 5 873 906

## Description

La présente invention concerne une structure d'une prothèse destinée à être implantée dans un conduit humain ou animal, ainsi qu'une prothèse pourvue d'une telle structure.

Une telle prothèse qui, dans le cadre de la présente invention, est destinée à assurer (c'est-à-dire à rétablir ou à préserver) un passage dans le conduit humain ou animal dans lequel elle est implantée, concerne plus particulièrement, bien que non exclusivement, une prothèse vasculaire, simple ou bifurquée.

On sait qu'une prothèse vasculaire de ce type peut être utilisée, en particulier :
a) pour rétablir un passage suffisant pour le sang dans une artère, en particulier en cas de sténose. La prothèse qui sert alors d'élargisseur de conduit ou d'artère comprend généralement une structure à mémoire de forme susceptible d'être amenée d'une position repliée, permettant l'implantation, à une position déployée dans l'artère, en vue d'élargir cette dernière ; ou
b) pour préserver un tel passage, notamment pour traiter un anévrisme. Dans ce cas, la prothèse comporte, en plus d'une structure telle que celle précitée, une enveloppe externe étanche entourant au moins partiellement ladite structure. La prothèse est implantée dans l'artère de sorte que cette enveloppe couvre complètement l'anévrisme et elle sert alors à véhiculer le sang dans cette partie fragilisée de l'artère.

De nombreux exemples de structures pour de telles prothèses sont connus. On peut citer, à titre d'exemples, les documents EP-0 691 108, FR-2 745 172, EP-0 880 948, EP-0 808 614, EP-0 808 612 ou WO-95/21592, EP-0 749 729.

Parmi ces documents, le document WO-95/21592 décrit notamment une structure de prothèse qui comprend un treillis sensiblement cylindrique. Ce treillis est constitué d'un fil qui est configuré de manière à former des anneaux ondulés qui sont liés entre eux de manière à obtenir ledit treillis cylindrique. A cet effet, au moins certaines des ondulations dudit fil ondulé, respectivement de deux anneaux adjacents, sont liées deux à deux par des moyens de liaison. Les moyens de liaison préférés par ce document connu sont des fils de suture.

D'autres types de moyens de liaison pour le même type de structure ou des structures similaires sont également connus. On connaît en particulier :
- des soudures (document FR-2 745 172);
- des agrafes (document WO-95/21592 précité) ; ou
- une réalisation en une seule pièce du treillis (fil et moyens de liaison).

Dans ce cas, le treillis peut notamment être obtenu par découpage de la matière dans une pièce en feuille (document WO-95/32688).

Il est évident que, pour permettre à la structure de remplir ses différentes fonctions, les moyens de liaison utilisés doivent notamment :
- assurer une liaison solide et permanente, toute rupture d'une telle liaison pouvant avoir des conséquences tragiques pour le patient ;
- présenter une grande souplesse, pour permettre et faciliter à la fois l'implantation et le fonctionnement de la prothèse ; et
- ne pas blesser le patient et en particulier la paroi interne du conduit, dans lequel la prothèse est implantée.

Toutefois, aucun des moyens de liaison connus ne permet de remplir simultanément toutes ces conditions. En effet :
- les soudures sont rigides et ne sont donc pas en mesure d'apporter la souplesse nécessaire à la structure. En outre, lorsque la structure est amenée dans la position repliée, en vue de l'implantation, il apparaît un risque de rupture au moins partielle de ces soudures, ce qui fragilise la structure. De plus, si une telle rupture a lieu, la structure peut comporter des aspérités susceptibles d'être blessantes pour la paroi artérielle ;
- les liaisons par découpage de matière sont elles aussi trop rigides pour assurer la souplesse nécessaire à la structure. De plus, la réalisation d'une telle structure est complexe; et
- les agrafes présentent une souplesse limitée, en particulier par le fait qu'elles coincent le fil à lier. De plus, la mise en place d'une agrafe est susceptible d'endommager le fil.

La présente invention a pour objet de remédier à ces inconvénients. Elle concerne une structure de prothèse du type précité, très sûre et très souple, pouvant par exemple être amenée facilement et en toute sécurité d'une position repliée, en vue de l'implantation dans une artère, à une position déployée de fonctionnement dans l'artère et pouvant être maintenue en toute sécurité dans cette position déployée.

A cet effet, selon l'invention, ladite structure qui comprend au moins un fil ondulé formant des unités sensiblement annulaires liées entre elles, au moins certaines des ondulations dudit fil ondulé respectivement de deux unités adjacentes étant liées deux à deux par des moyens de liaison, est remarquable en ce qu'au moins certains desdits moyens de liaison comportent des maillons qui sont réalisés en une pièce rigide et qui sont munis au moins de deux boucles solidaires l'une de l'autre, et en ce que, pour chacun desdits maillons, chacune des deux boucles dudit maillon emprisonne, avec du jeu, respectivement l'une des deux ondulations à lier entre elles, lesdites deux ondulations (ON) ainsi liées étant indépendantes l'une de l'autre.

Ainsi, grâce à l'invention :
- comme les moyens de liaison comprennent des maillons qui sont réalisés en une pièce rigide et qui comprennent des boucles emprisonnant les ondulations du fil, les maillons rigides ne peuvent pas rompre sous les pressions susceptibles d'être exercées sur la structure et les ondulations ne peuvent pas s'échapper desdites boucles de sorte que la liaison ainsi obtenue est très solide et durable ;
- comme les ondulations sont emprisonnées avec un certain jeu dans les boucles, elles peuvent se mouvoir aisément, ce qui confère une grande souplesse à la structure. Ceci permet notamment d'éviter des plis permanents existant souvent avec les moyens de liaison connus (soudure, ...) suite au pliage de la structure entre les différentes positions possibles, de tels plis permanents étant susceptibles de réduire la surface de la section transversale du canal que l'on veut créer avec la prothèse implantée ; et
- comme chaque maillon comporte au moins deux boucles, c'est-à-dire une boucle par ondulation ou partie de fil à lier, les parties de fils ainsi liées sont indépendantes l'une de l'autre, ce qui améliore la souplesse de la structure, d'une part en évitant les frottements entre ces parties de fil et, d'autre part, en séparant l'une de l'autre les deux parties de la structure associées respectivement à ces deux parties de fil.

On notera que, dans le cadre de la présente invention, au moins certains des maillons peuvent comporter plus de deux boucles, ce qui permet de lier ensemble simultanément plus de deux ondulations ou parties de fil. Un tel mode de réalisation peut, par exemple, être intéressant pour la fixation des mailles de la structure au niveau de bifurcations ou au niveau d'un changement de diamètre du treillis cylindrique.

Dans un premier mode de réalisation préféré, au moins l'un desdits maillons comporte au moins :
- un tronçon central rectiligne ; et
- à chacune des extrémités dudit tronçon central, au moins un tronçon en arc de cercle destiné à former au moins une partie d'une boucle du maillon.

Dans un second mode de réalisation, au moins l'un desdits maillons comporte au moins :
- un tronçon central comprenant deux tronçons partiels rectilignes, non alignés et raccordés entre eux ; et
- à l'extrémité libre de chacun desdits tronçons partiels, au moins un tronçon en arc de cercle destiné à former au moins une partie d'une boucle du maillon.

Par ailleurs, de préférence, au moins l'un desdits maillons présente une forme générale en forme de S, définie dans un seul plan.

Toutefois, ceci n'est pas la seule réalisation possible. En effet, il est envisageable, dans le cadre de la présente invention, que, pour au moins l'un desdits maillons, l'une des deux boucles dudit maillon est définie ou située dans un premier plan qui est différent d'un second plan dans lequel est définie ou située l'autre boucle du maillon.

En outre, selon l'invention :
- dans une première variante, au moins l'une des boucles d'au moins l'un desdits maillons est entièrement fermée, ce qui assure une attache extrêmement solide ; et
- dans une seconde variante, au moins l'une des boucles d'au moins l'un desdits maillons est partiellement fermée de manière à pouvoir emprisonner l'ondulation à lier, ce qui facilite la mise en place du maillon, puisque, lors de cette mise en place, il n'est pas nécessaire d'exercer une force pour complètement fermer la boucle.

Par ailleurs, dans le cadre de la présente invention, le treillis et notamment ses mailles peuvent présenter différentes formes. En particulier, avantageusement :
- dans un premier mode de réalisation, au moins certaines desdites ondulations sont des zigzags ; et
- dans un second mode de réalisation, ledit treillis comporte, au moins partiellement, des mailles hexagonales, de manière à obtenir une forme dite de "nid-d'abeilles".

En outre, de façon avantageuse, au moins l'un desdits maillons est radio-opaque, ce qui permet notamment de pouvoir facilement détecter et situer, par radiographie, la structure conforme à l'invention, lors de son implantation ou après son implantation dans le corps d'un patient. Pour améliorer cette détection, ladite structure comporte avantageusement une pluralité de maillons radio-opaques disposés longitudinalement audit treillis cylindrique.

La présente invention concerne également une prothèse destinée à être implantée dans un conduit humain ou animal pour assurer un passage dans ledit conduit. Selon l'invention, ladite prothèse, par exemple une prothèse tubulaire simple ou une prothèse bifurquée, comporte au moins une structure telle que celle précitée. En fonction des applications envisagées (traitement d'un anévrisme par exemple), elle peut également comporter au moins une enveloppe étanche entourant, extérieurement et/ou intérieurement, au moins en partie, ladite structure.

De plus de préférence, ladite enveloppe étanche présente un retour à au moins l'une des extrémités de ladite structure, ce qui permet d'assurer une bonne étanchéité à cette extrémité dans le cas de son assemblage à une autre prothèse, par exemple dans le cas de l'assemblage de jambages aux jambes d'une prothèse bifurquée.

Les figures du dessin annexé feront bien comprendre comment l'invention peut être réalisée. Sur ces figures, des références identiques désignent des éléments semblables.

La figure 1 illustre schématiquement une partie d'une structure de prothèse conforme à l'invention.

Les figures 2 et 3 montrent un premier mode de réalisation d'un maillon conforme à l'invention, respectivement dans des positions fermée et ouverte.

La figure 4 montre un second mode de réalisation d'un maillon conforme à l'invention.

Les figures 5 et 6 montrent respectivement deux modes de réalisations de mailles d'une structure conforme à l'invention.

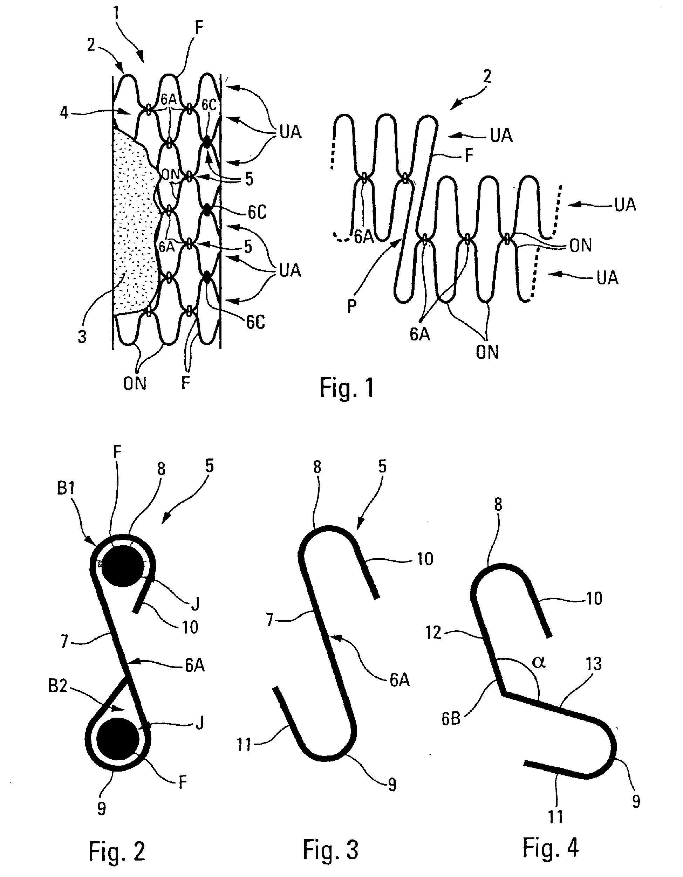

La prothèse 1 conforme à l'invention et représentée schématiquement sur la figure 1 est destinée à être implantée dans un conduit humain ou animal, notamment dans une artère, pour assurer, c'est-à-dire préserver ou rétablir, un passage dans ledit conduit.

Bien que non exclusivement, cette prothèse 1 est destinée plus particulièrement à traiter des altérations physiques, comme par exemple des anévrismes ou des sténoses, d'artères du corps humain.

A cet effet, en fonction des altérations traitées, cette prothèse peut être simplement de forme tubulaire ou peut présenter une forme plus complexe, par exemple une forme bifurquée, adaptée notamment à la forme de l'artère dans laquelle elle doit être implantée.

Une telle prothèse 1 comporte au moins une structure 2 conforme à l'invention et précisée ci-dessous. Elle peut être munie d'au moins une enveloppe externe étanche 3 connue, qui recouvre au moins en partie ladite structure 2 et qui est représentée, de façon partiellement arrachée, sur la figure 1. Ladite enveloppe peut être interne et/ou externe à la structure.

A titre d'application préférée, mais non exclusive, une telle prothèse 1 :
- lorsqu'elle est munie d'une enveloppe 3, est appropriée au traitement d'anévrismes ; et
- lorsqu'elle ne comporte pas une telle enveloppe, peut être utilisée comme élargisseur de conduit ou d'artère, en particulier pour le traitement de sténoses.

La structure 2 est réalisée en un matériau biocompatible à mémoire de forme de manière à pouvoir être amenée d'une position repliée et compacte, pour permettre son implantation dans le conduit considéré, dans une position déployée, lorsqu'elle est en place dans ledit conduit.

Comme on peut le voir sur la partie gauche de la figure 1, ladite structure 2 comprend, de façon connue, au moins un treillis 4 qui est au moins en partie cylindrique et qui comporte au moins un fil ondulé F formant des unités ondulés UA sensiblement annulaires, liées entre elles. Ces unités ondulées UA comportent une pluralité d'ondulations ON. De plus, au moins certaines de ces ondulations ON, à chaque fois de deux unités UA adjacentes, sont liées deux à deux par des moyens de liaison 5, de manière à relier ensemble lesdites unités UA adjacentes et à ainsi former ledit treillis cylindrique 4.

Dans le cadre de la présente invention, chaque unité annulaire UA peut être réalisée au moyen d'un fil F spécifique de sorte que les différentes unités annulaires UA sont alors, avant liaison, complètement indépendantes les unes des autres.

Toutefois, il est également possible de réaliser l'ensemble des unités annulaires UA avec un seul et même fil F, comme représenté partiellement sur la partie droite de la figure 1. A cet effet, le fil F est configuré de manière à passer, après création d'une unité annulaire UA, à l'unité annulaire suivante UA, comme représenté par exemple en P.

Selon l'invention, notamment pour permettre une liaison solide, souple, durable et non blessante pour la paroi du conduit dans lequel est implantée la prothèse 1, au moins certains des moyens de liaison 5 comportent des maillons 6A, 6B, 6C conformes à la présente invention et représentés sur les figures 1 à 6.

Selon l'invention, un tel maillon 6A, 6B, 6C est réalisé dans une pièce rigide et comporte au moins deux boucles B1 et B2, dont chacune emprisonne, avec un jeu J, l'une des ondulations ou parties de fil F à lier ensemble, comme on peut le voir sur la figure 2. Les boucles B1 et B2 sont telles que les parties de fil (emprisonnées avec jeu), tout en étant rendues solidaires, peuvent se mouvoir (tourner) librement, ce qui permet d'obtenir une liaison présentant les caractéristiques précitées.

En effet :
- la liaison est solide et durable, grâce à la rigidité du maillon 6A et à la génération de boucles B1 et B2 dans la pièce même du maillon 6A ;
- la liaison est très souple, en particulier grâce au jeu J et à la séparation des deux parties de fil F devant être liées l'une à l'autre ;
- la liaison ne comprend, par exemple, aucune partie saillante et il n'existe donc aucun danger de blesser la paroi du conduit ou de l'artère, dans lequel est implantée la prothèse 1.

Dans le cadre de la présente invention, le fil F et les maillons 6A, 6B et 6C peuvent être réalisés en métal ou par exemple en un matériau connu commercialisé sous le nom de "nitinol".

Dans un premier mode de réalisation préféré représenté sur les figures 2 et 3, le maillon 6A comporte :
- un tronçon central rectiligne 7 ;
- à chacune des extrémités dudit tronçon central 7, un tronçon 8, 9 en arc de cercle ; et
- à chacune des extrémités desdits tronçons 8, 9, un tronçon d'extrémité 10, 11 rectiligne.

Comme on peut le voir sur les figures 2 et 3, la figure 2 correspondant à une position fermée ou position de liaison du maillon 6A et la figure 3 illustrant la position ouverte de ce maillon 6A avant la liaison, les tronçons 8 et 10 et les tronçons 9 et 11 sont recourbés autour des parties de fil F ou ondulations, pour former respectivement les boucles B1 et B2.

Dans le cadre de la présente invention :
- les boucles, comme la boucle B2 de la figure 2, peuvent être complètement fermées, ce qui assure une liaison extrêmement solide ;
- lesdites boucles peuvent également n'être que partiellement fermées, c'est-à-dire juste suffisamment pour emprisonner efficacement la partie de fil ou ondulation à lier, comme cela est le cas pour la boucle B1 de la figure 2, ce qui facilite la réalisation des boucles.

Le choix de l'une ou l'autre des solutions précitées peut dépendre notamment des propriétés du matériau utilisé pour réaliser le maillon 6A, et en particulier de ses propriétés de rigidité.

Bien entendu, la présence de deux boucles B1 et B2 de types différents sur le même maillon 6A est due essentiellement à des raisons de simplification du dessin. Généralement, mais non exclusivement, les boucles d'un même maillon sont en effet du même type.

Dans un second mode de réalisation représenté sur la figure 4, le maillon 6B comporte (au lieu d'un tronçon central rectiligne 7 comme sur la figure 2) au moins deux tronçons partiels rectilignes 12 et 13 raccordés entre eux de manière à former un angle α.

Ce mode de réalisation permet notamment de rapprocher davantage les boucles obtenues dans la position de liaison, par rapport au mode de réalisation des figures 2 et 3. Ce rapprochement, qui est inversement proportionnel à la valeur de cet angle (aigu) α, peut être intéressant pour certaines parties de certains types de structures, en particulier pour la bifurcation d'une structure bifurquée.

Dans ce dernier mode de réalisation, en variante, les tronçons 12 et 13 peuvent être raccordés entre eux, non pas directement, mais par l'intermédiaire d'au moins un tronçon supplémentaire non représenté.

De préférence, comme représenté sur les figures 2 à 4, les maillons 6A, 6B conformes à l'invention présentent une forme générale en S, définie dans un seul plan.

Toutefois, dans un autre mode de réalisation non représenté, l'une des boucles d'un maillon peut être définie ou située dans un premier plan qui est différent d'un second plan dans lequel est définie ou située une autre ou l'autre boucle de ce maillon.

Par ailleurs, pour permettre de détecter et donc de situer exactement la structure 2 lors de son implantation dans un corps humain ou animal ou après cette implantation, ladite structure 2 comporte des maillons 6C conformes à l'invention et illustrés en trait épais sur la figure 1, qui sont radio-opaques et donc détectables par radiographie. Cette caractéristique particulière peut être obtenue par l'utilisation d'un matériau approprié ou le recouvrement du maillon 6C par un revêtement approprié.

De plus, pour permettre une détection précise du positionnement de la structure 2, cette dernière comporte dans ce cas, avantageusement, une pluralité de maillons opaques 6C situés longitudinalement au treillis tubulaire 4, par exemple un maillon par unité annulaire UA.

Dans le cadre de la présente invention, le treillis 4 peut comporter des mailles M1, M2 et des ondulations ON de différentes formes.

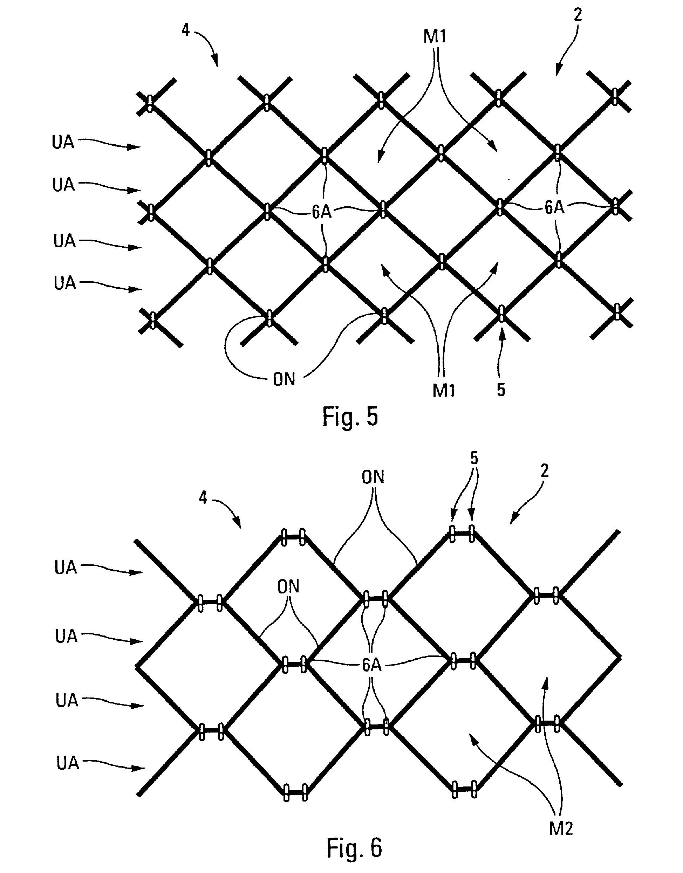

En particulier, les ondulations ON peuvent notamment être :
- des sinusoïdes, comme représenté sur la figure 1 ; ou
- des zigzags, comme représenté sur la figure 5.

De plus, les mailles du treillis 4 peuvent notamment présenter :
- une forme carrée, comme les mailles M1 représentées sur la figure 5 ; ou
- une forme hexagonale ou en "nid-d'abeilles", comme les mailles M2 représentées sur la figure 6.

## Revendications

1. Structure d'une prothèse destinée à être implantée dans un conduit humain ou animal pour assurer un passage dans ledit conduit, ladite structure (2) comprenant au moins un treillis (4) qui est, au moins en partie, sensiblement cylindrique et qui comporte au moins un fil ondulé (F) formant des unités (UA) sensiblement annulaires liées entre elles, au moins certaines des ondulations (ON) dudit fil ondulé (F) respectivement de deux unités (UA) adjacentes étant liées deux à deux par des moyens de liaison (5),
**caractérisée en ce qu'**au moins certains desdits moyens de liaison (5) comportent des maillons (6A, 6B, 6C) qui sont réalisés en une pièce rigide et qui sont munis au moins de deux boucles (B1, B2) solidaires l'une de l'autre, et **en ce que**, pour chacun desdits maillons (6A, 6B, 6C), chacune des deux boucles (B1, B2) dudit maillon (6A, 6B, 6C) emprisonne avec du jeu (J) respectivement l'une des deux ondulations (ON) à lier entre elles, lesdites deux ondulations (ON) ainsi liées étant indépendantes l'une de l'autre.

2. Structure selon la revendication 1,
**caractérisée en ce qu'**au moins l'un desdits maillons (6A) comporte au moins :
- un tronçon central (7) rectiligne ; et
- à chacune des extrémités dudit tronçon central (7), au moins un tronçon (8, 9) en arc de cercle destiné à former au moins une partie d'une boucle (B1, B2) du maillon (6A).

3. Structure selon la revendication 1,
**caractérisée en ce qu'**au moins l'un desdits maillons (6B) comporte au moins :
- un tronçon central comprenant deux tronçons partiels (12, 13) rectilignes, non alignés et raccordés entre eux ; et
- à l'extrémité libre de chacun desdits tronçons partiels (12, 13), au moins un tronçon (8, 9) en arc de cercle destiné à former au moins une partie d'une boucle du maillon (6B).

4. Structure selon l'une quelconque des revendications 1 à 3,
**caractérisée en ce qu'**au moins l'un desdits maillons (6A, 6B) présente une forme générale en forme de S, définie dans un seul plan.

5. Structure selon l'une des revendications 1 et 3,
**caractérisée en ce que**, pour au moins l'un desdits maillons, l'une des deux boucles dudit maillon est définie dans un premier plan qui est différent d'un second plan dans lequel est définie l'autre boucle du maillon.

6. Structure selon l'une quelconque des revendications précédentes,
**caractérisée en ce qu'**au moins l'une (B2) des boucles d'au moins l'un desdits maillons (6A) est entièrement fermée.

7. Structure selon l'une quelconque des revendications 1 à 6,
**caractérisée en ce qu'**au moins l'une (B1) des boucles d'au moins l'un desdits maillons (6A) est partiellement fermée de manière à emprisonner l'ondulation (ON) à lier.

8. Structure selon l'une quelconque des revendications 1 à 7,
**caractérisée en ce qu'**au moins certaines desdites ondulations (ON) sont des zigzags.

9. Structure selon l'une quelconque des revendications 1 à 8,
**caractérisée en ce que** ledit treillis comporte, au moins partiellement, des mailles hexagonales (M2).

10. Structure selon l'une quelconque des revendications précédentes,
**caractérisée en ce qu'**au moins l'un desdits maillons (6C) est radio-opaque.

11. Structure selon la revendication 10,
**caractérisée en ce qu'**elle comporte une pluralité de maillons radio-opaques (6C) disposés longitudinalement audit treillis cylindrique (4).

12. Prothèse destinée à être implantée dans un conduit humain ou animal pour assurer un passage dans ledit conduit,
**caractérisée en ce qu'**elle comprend au moins une structure (2) telle que celle spécifiée sous l'une quelconque des revendications 1 à 11.

13. Prothèse selon la revendication 12,
**caractérisée en ce qu'**elle comporte de plus au moins une enveloppe étanche (3) entourant, au moins en partie, ladite structure (2).

14. Prothèse selon la revendication 13,
**caractérisée en ce que** ladite enveloppe étanche (3) présente un retour à au moins l'une des extrémités de ladite structure (2).

## Claims

1. A structure of a prosthesis intended to be implanted in a human or animal passage to provide through-passage along said passage, said structure (2) comprising at least one mesh (4) which, at least in part, is approximately cylindrical and comprises at least one corrugated filament (F) forming approximately annular units (UA) linked together, at least some of the corrugations (ON) of said corrugated filament (F) of two respective adjacent units (UA) being linked together by linking means (5),
**characterized in that** at least some of said linking means (5) comprise links (6A, 6B, 6C) which are made as a rigid piece and which are provided with at least two loops (B1, B2) joined together and,**in that**, in the case of each of said links (6A, 6B, 6C), each of the two loops (B1, B2) of said link (6A, 6B, 6C) entraps, with some clearance (J), a respective one of the two corrugations (ON) which are to be linked together, said two corrugations (ON) thus linked are independent of one another.

2. The structure as claimed in claim 1,
**characterized in that** at least one of said links (6A) comprises at least:
- a straight central portion (7); and
- at each of the ends of said central portion (7), at least one portion (8, 9) in the shape of an arc of a circle intended to form at least part of a loop (B1, B2) of the link (6A).

3. The structure as claimed in claim 1,
**characterized in that** at least one of said links (6B) comprises at least:
- a central portion comprising two straight partial portions (12, 13) which are not aligned and which are connected together; and
- at the free end of each of said partial portions (12, 13), at least one portion (8, 9) in the shape of an arc of a circle intended to form at least part of one loop of the link (6B).

4. The structure as claimed in any one of claims 1 to 3,
**characterized in that** at least one of said links (6A, 6B) has the overall shape of an S, defined in a single plane.

5. The structure as claimed in any one of claims 1 to 3,
**characterized in that**, in the case of at least one of said links, one of the two loops of said link is defined in a first plane which differs from a second plane in which the other loop of the link is defined.

6. The structure as claimed in any one of the preceding claims,
**characterized in that** at least one (B2) of the loops at least one of said links (6A) is entirely closed.

7. The structure as claimed in any one of claims 1 to 6,
**characterized in that** at least one (B1) of the loops of at least one of said links (6A) is partially closed so as to entrap the corrugation (ON) that is to be linked.

8. The structure as claimed in any one of claims 1 to 7,
**characterized in that** at least some of said corrugations (ON) are zigzags.

9. The structure as claimed in any one of claims 1 to 8,
**characterized in that** said mesh at least partially comprises hexagonal mesh openings (M2).

10. The structure as claimed in any one of the preceding claims,
**characterized in that** at least one of said links (6C) is radio-opaque.

11. The structure as claimed in claim 10,
**characterized in that** it comprises a number of radio-opaque links (6C) arranged longitudinally with respect to said cylindrical mesh (4).

12. A prosthesis intended to be implanted in a human or animal passage to provide through-passage along said passage,
**characterized in that** it comprises at least one structure (2) as specified in any one of claims 1 to 11.

13. The prosthesis as claimed in claim 12,
**characterized in that** it additionally comprises at least one impervious envelope (3) at least partially surrounding said structure (2).

14. The prosthesis as claimed in claim 13,
**characterized in that** said impervious envelope (3) has a turned-back region at least at one of the ends of said structure (2).

## Patentansprüche

1. Prothesenanordnung zur Implantation in ein menschliches oder tierisches Gefäß, um einen Durchgang in dem Gefäß zu gewährleisten, wobei die Anordnung (2) mindestens ein Geflecht- (4) umfasst, das mindestens teilweise im Wesentlichen zylindrisch ist und das mindestens eine wellige Faser (F) umfasst, die im Wesentlichen ringförmige, untereinander verbundene Einheiten (UA) bildet, wobei mindestens einzelne der Welligkeiten (ON) der welligen Faser (F) von jeweils zwei aneinandergrenzenden Einheiten (UA) durch Verbindungsmittel (5) zwei zu zwei verbunden sind,
**dadurch gekennzeichnet, dass** mindestens einzelne der Verbindungsmittel (5) Glieder (6A, 6B, 6C) umfassen, die aus einem starren Teil gebildet sind und die mit mindestens zwei Schleifen (B1, B2) versehen sind, die fest miteinander verbunden sind, und dass bei jedem der Glieder (6A, 6B, 6C) jede der beiden Schleifen (B1, B2) des Gliedes (6A, 6B, 6C) mit Spiel (J) jeweils eine der beiden miteinander zu verbindenden Welligkeiten (ON) einschließt, wobei die auf diese Weise verbundenen Welligkeiten (ON) voneinander unabhängig sind.

2. Anordnung nach Anspruch 1,
**dadurch gekennzeichnet, dass** mindestens eines der Glieder (6A) mindestens Folgendes umfasst:
- einen geraden mittleren Abschnitt (7); und
- an jedem der Enden des mittleren Abschnitts (7) mindestens einen kreisbogenförmigen Abschnitt (8, 9), der dazu bestimmt ist, mindestens einen Teil einer Schleife (B1, B2) des Gliedes (6A) zu bilden.

3. Anordnung nach Anspruch 1,
**dadurch gekennzeichnet, dass** mindestens eines der Glieder (6B) mindestens Folgendes umfasst:
- einen mittleren Abschnitt mit zwei geraden, nicht auf einer Linie befindlichen und miteinander verbundenen Teilabschnitten (12, 13); und
- am freien Ende jedes der Teilabschnitte (12, 13) mindestens einen kreisbogenförmigen Abschnitt (8, 9), der dazu bestimmt ist, mindestens einen Teil einer Schleife des Gliedes (6B) zu bilden.

4. Anordnung nach einem der Ansprüche 1 bis 3,
**dadurch gekennzeichnet, dass** mindestens eines der Glieder (6A, 6B) als allgemeine Form eine S-Form aufweist, die in einer einzigen Ebene bestimmt ist.

5. Anordnung nach einem der Ansprüche 1 bis 3,
**dadurch gekennzeichnet, dass** bei mindestens einem der Glieder eine der Schleifen des Gliedes in einer ersten Ebene bestimmt ist, die sich von einer zweiten Ebene unterscheidet, in der die- andere Schleife des Gliedes bestimmt ist.

6. Anordnung nach einem-der vorhergehenden Ansprüche,
**dadurch gekennzeichnet, dass** mindestens eine (B2) der Schleifen von mindestens einem der -Glieder (6A) zur Gänze geschlossen ist.

7. Anordnung nach einem der Ansprüche 1 bis 6,
**dadurch gekennzeichnet, dass** mindestens eine (B1) der Schleifen von mindestens einem der Glieder (6A) teilweise geschlossen ist, um die zu verbindende Welligkeit- (ON) einzuschließen.

8. Anordnung nach einem der Ansprüche 1 bis 7,
**dadurch gekennzeichnet, dass** mindestens einzelne der Welligkeiten (ON) zickzackförmig sind.

9. Anordnung nach einem der Ansprüche 1 bis 8,
**dadurch gekennzeichnet, dass** das Geflecht mindestens teilweise sechseckige Maschen (M2) umfasst.

10. Anordnung nach einem der vorhergehenden Ansprüche,
**dadurch gekennzeichnet, dass** mindestens eines der Glieder (6C) röntgendicht ist.

11. Anordnung nach Anspruch 10,
**dadurch gekennzeichnet, dass** sie eine Vielzahl von röntgendichten Gliedern (6C) umfasst, die in Längsrichtung in dem zylindrischen Geflecht (4) angeordnet sind.

12. Prothese zur Implantation in ein menschliches oder tierisches Gefäß, um einen Durchgang in dem Gefäß zu gewährleisten,
**dadurch gekennzeichnet, dass** sie mindestens eine Anordnung (2) umfasst, wie sie in einem der Ansprüche 1 bis 11 spezifiziert ist.

13. Prothese nach Anspruch 12,
**dadurch gekennzeichnet, dass** sie darüber hinaus mindestens eine dichte Umhüllung (3-) umfasst, die die Anordnung (2) mindestens teilweise umgibt.

14. Prothese nach Anspruch- 13,
**dadurch gekennzeichnet, dass** die dichte Umhüllung (3) an mindestens einem der Enden der Struktur (2) eine Umstülpung umfasst.
